# EUROPEAN PATENT SPECIFICATION

(11) **EP 0 806 425 B1**
(45) Date of publication and mention of the grant of the patent: **29.08.2001**
(21) Application number: 96302472.4
(22) Date of filing: 09.04.1996
(51) Int. Cl.: C07D 473/00

(54) **An improved regiospecific process for synthesis of acyclic nucleosides**
Verbessertes regiospezifisches Verfahren zur Herstellung von azyklische Nucleosiden
Procédé régiospécifique amélioré pour la synthèse de nucléosides acycliques

(43) Date of publication of application: 12.11.1997
(60) Divisional application: 99116909.5
(73) Proprietor: LUPIN LABORATORIES LIMITED, Bombay, Maharashtra - 400098 (IN)
(72) Inventor: Kumar, Ashoke, Dr., Mandideep, Dist. Raisen, M. P. 462 046 (IN); Singh, Dharmendra, Mandideep, Dist. Raisen, M. P. 462 046 (IN); Wani, Mukesh Jagannath, Mandideep, Dist. Raisen, M. P. 462 046 (IN); Joshi, Narendra Sriram, Mandideep, Dist. Raisen, M. P. 462 046 (IN); Thombre, Pravin Sahadev, Mandideep, Dist. Raisen, M. P. 462 046 (IN); Rawat, Ajay Singh, Mandideep, Dist. Raisen, M. P. 462 046 (IN)
(74) Representative: Thornley, Rachel Mary

(56) References cited:
- EP-A- 0 184 473
- EP-A- 0 532 878
- EP-A- 0 704 445
- WO-A-95/07281
- JP-A- 59 080 685
- US-A- 4 199 574
- US-A- 4 355 032
- CHEMICAL & PHARMACEUTICAL BULLETIN, vol. 36, no. 3, March 1988, pages 1153-1157, XP002011413 HIROATSU MATSUMOTO ET AL:
- The Merck Index, Twelfth Edition, 1996, page 9678
- R. Zou et al., Can. J. Chem., 65, (1982), p.1436-1437

## Description

This invention relates to an improved regiospecific process for the synthesis of acyclic nucleosides such as acyclovir and ganciclovir, anti-viral compounds especially effective against herpes virus, and intermediates thereof starting from diacylguanine and appropriate addendum, selected from 2-oxa-1,4-butanediol diacetate (OBDDA), 1,4-diacetoxy-3-acetoxymethyl-2-oxa-butane, 1,4-dibenzyloxy-3-acetoxymethyl-2-oxabutane.

Both acyclovir (Ia) and ganciclovir (Ib) show remarkable anti-viral activities (USP 4 199 574, USP 4 355 032) 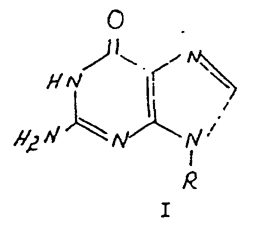
Ia : R = -CH₂-O-CH₂CH₂-OH (Acyclovir)
Ib : R = CH₂-O-CH(CH₂OH)CH₂OH (Ganciclovir)

The strategy adopted in the prior art for manufacture of I is alkylation of appropriately substituted 2-aminopurines e.g. guanine derivatives with required addendum which essentially includes an acid with or without solvent to yield N-9 alkylated intermediate (II) e.g. N²-acetyl-9-[(2-acetoxy ethoxy)methyl] quanine (IIa) along with corresponding N-7 alkylated isomer (IIIa). In particular, it is known to react mono or diacetylated guanine with 2-oxa-1,4-butanediol diacetate (OBDDA) to yield the intermediate compound of formula II. 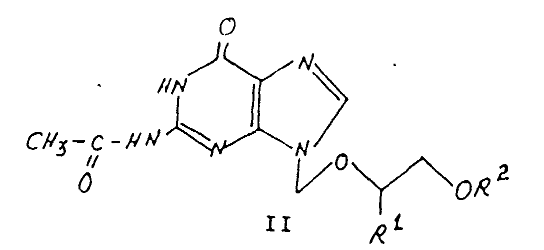
IIa : R¹ = H, R² = COCH₃
IIb : R¹ = CH₂OCH₂Ph, R² = CH₂Ph
IIc : R¹ = CH₂OCOCH₃, R² = COCH₃
alongwith compound of formula III

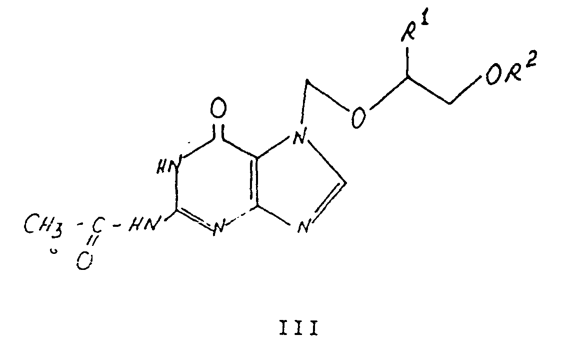

Wherein the formula III includes the compound according to IIIa, IIIb and IIIc, with R¹ and R² as defined in formula IIa, IIb and IIc respectively.

This intermediate mixture of II and III is purified generally by costly and tedious processes to remove the last traces of N-7 isomer(III) and then hydrolysed to yield I. Often N-7 isomer(IV) is associated with final product which is further removed employing a number of operations.

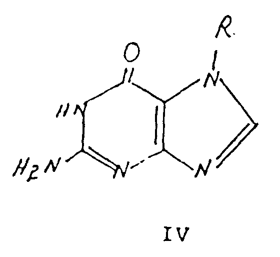

Wherein for IVa and IVb, R is as defined in formula I.

Since alkylation of guanine derivatives like diacetyl/monoacetyl guanine (DAG/MAG) of formula V and VT 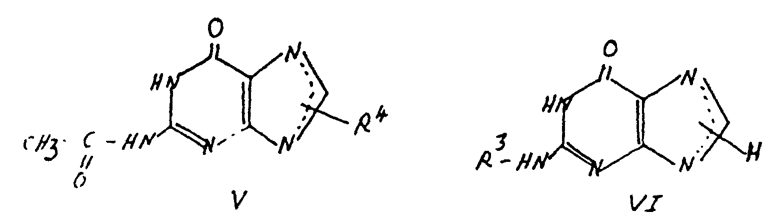 (Va: R⁴ = COCH₃, DAG) (VIa : R³ = COCH₃, MAG) wherein 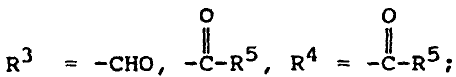 where R⁵ = methyl, ethyl, isopropyl, phenyl;
in presence of an acid, is a thermodynamically controlled reaction, N-7 isomer of formula III is always formed. However, N-9 isomer (II) being thermodynamically more stable (Chem Pharm Bull, 1970, 18, 1446) is produced as the major product.

The formation of N-7 isomer (III) increases the total cost of manufacture. Hence, there is a need for development of regiospecific process for the manufacture of II, the penultimate intermediate for I.

Some of the important methods for manufacture of acyclovir/ganciclovir of formula I reported in the prior art are described below:
1. BE 833 006, USP 4 199 574 describe a process for the synthesis of Acyclovir (Ia) which involves condensation of trimethylsilylated guanine with 2-benzoyloxyethoxy methyl chloride in DMF in the presence of a base followed by deprotection, yielding in the desired N-9 isomer(IIa) along with unacceptable amounts of its corresponding N-7 isomer(IIIa). The former after purification and deacetylation gives acyclovir in 24% overall yield. EP-A-0704445 teaches the acid catalysed alkylation of silylated derivatives of guanine to preferentially generate the N-9 isomer.
2. H. Matsumoto in Chem Pharm Bull, 36(3), 1153 - 1157 and JP 63-107982 teaches a process for the synthesis of acyclovir(Ia) by condensation of diacetyl guanine (DAG Va) with 2-oxa-1,4-butanediol diacetate (OBDDA, VIIa) in DMSO in presence of an acid catalyst to get a mixture of N-9/N-7 isomer (66:26). The former is isolated by column chromatography and deacetylated with methanolic ammonia to qive acyclovir. Overall yield of acyclovir from guanine is 42%
   The process is illustrated in the following scheme A: 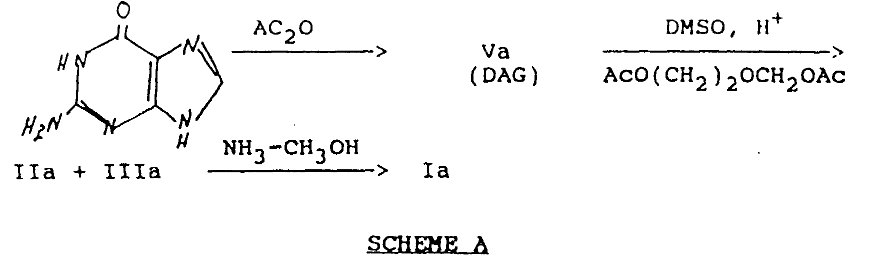
3. JP 59-80685 utilises similar chemistry but starts from N²-monoacetyl guanine (MAG, VIb) in presence or absence of solvent to yield a mixture of N-9 and N-7 alkylated guanine derivatives (N-9/N-7 ratio 52 : 26). The former subsequently isolated and deprotected to give acyclovir in overall 43 % yield.
4. EP 532 878 describes a process in which a a transgly cosilation reaction between guanosine, acetic anhydride and 2-oxa-1,4-butanediol diacetate (OBDDA) in the presence of catalytic amount of an acid is carried out, followed by hydrolysis to yield a mixture of acyclovir (Ia) and its N-7 isomer (IVa). The above process is illustrated in the following scheme B:

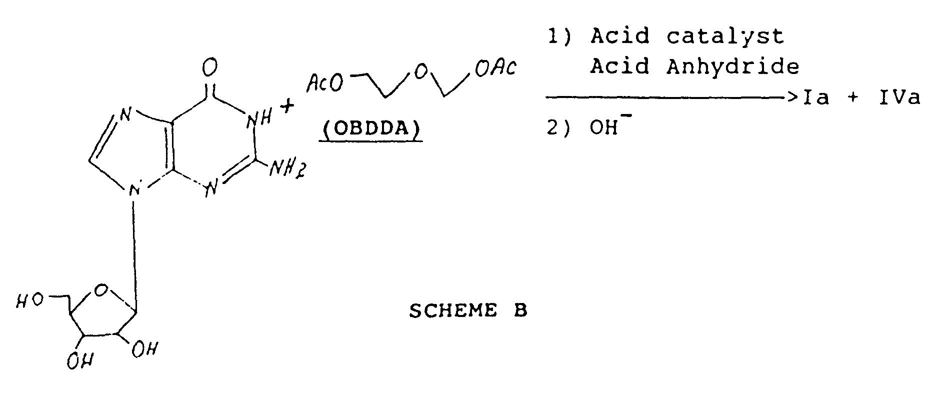

It can be observed that the major drawback in all the processes described above is that acyclovir (Ia) or its intermediate (IIa) is always contaminated with substantial amount of its N-7 isomer, and hence the separation of the desired N-9 isomer from the mixture is very tedious and requires chromatographic separation or fractional crystallisation.

In addition to the above, the PCT patent specification WO 95/027281 describes a process for the synthesis of Acyclovir (Ia) from N²-formylguanine. The chemistry of the process is illustrated in the following Scheme C: 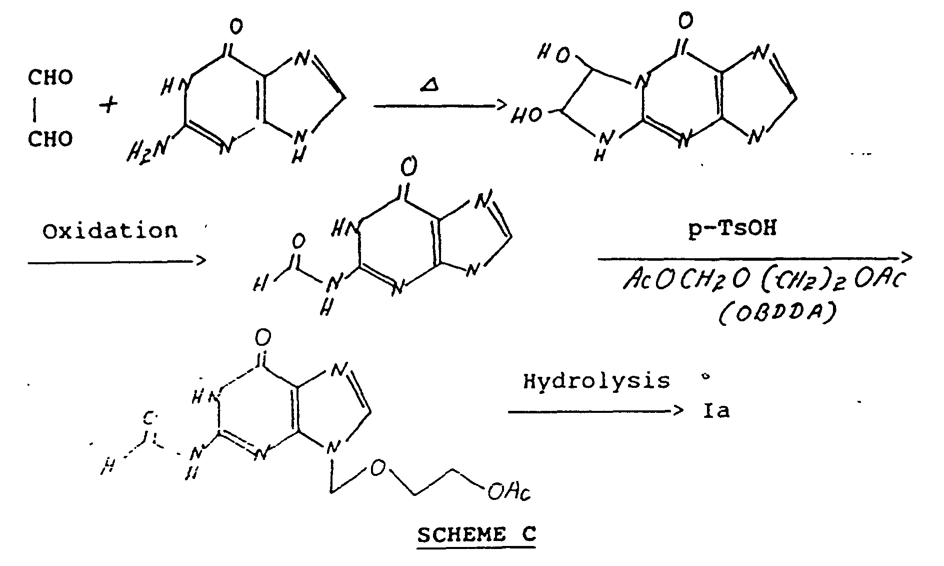 As illustrated in the above scheme, acyclovir is synthesised in four steps:
1) condensation reaction between quanine and glyoxal,
2) oxidation of the resulting tricyclic vicinal diol to N²-formyl guanine
3) alkylation of N²-formylguanine with OBDDA in the presence of an acid,
4) hydrolysis of N²-formyl-N⁹alkylated guanine to Acyclovir.

The final crude product is subjected to elaborate purification steps to get guanine free acyclovir of pharmaceutical grade. It is a lengthy process and hence not practical.

EP-A-0184473 teaches a regioselective synthesis of N-9 substituted guanine derivatives in which a blocking group at the 6-position minimises the formation of the N-7 substituted isomer. Acid catalysis is used for the alkylation reaction.

It might be highlighted that in the prior art, alkylation of DAG (Va), N²-acetyl guanine(MAG, Via) or N²-formylguanine to the penultimate intermediate of I is always an acid catalysed one; for instance p-toluenesulfonic acid is a common acid although other acids have also been employed [Chem Pharm Bull, 36(3), 1153-1157 (1988)]. Also use of a solvent is preferred in almost all the cases and no attempt has been made for recycle of the undesired N-7 isomer or developing conditions which can lead to selective formation of the desired isomer i.e II.

Prior art also does not specify the molar ratio of acid catalyst and alkylating agent with respect to protected guanine derivatives for obtaining high N-9/N-7 isomer ratio.

Accordingly, the basic objective of the present invention is to provide for a simple and cost effective_regiospecific process for the synthesis of N-9 alkylated guanine derivatives of general formula II such as N²-acetyl-9-[2-(acetoxyethoxy)methyl]guanine(IIa), N²-acetyl-9-[1,3-bis(benzyloxy-2-propoxy)methyl)guanine(IIb), which would be suitable intermediates for the manufacture of acyclic nucleosides of formula I such as acyclovir and ganciclovir having desired characteristics for its therapeutic use as anti-viral agents.

Other objects will be apparent from the description of the invention given herein below.

In accordance with the above basic objectives of the present invention in an aspect of the invention there is provided a regiospecific process for the manufacture of intermediate compound of formula I for use in synthesis of acyclic nucleocides of formula I which comprises reacting a substituted guanine derivative of formula V, 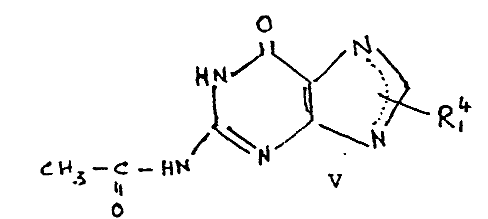 wherein 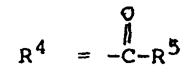 where R⁵ = methyl, ethyl, isopropyl, phenyl with alkylating agent of formula VII, 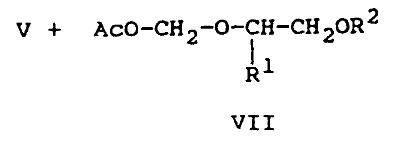 wherein R¹ and R² is as defined in formula II, without the presence of any acid catalyst and/or solvent under modified conditions comprising carrying out said reaction between protected guanine derivative and said alkalyting agent in the molar ratio of 1.5 to 6.0 preferably 1.5 to 2.5 at a temperature ranging from 90°-170° preferably between 100°C - 110 C for a period of 75-80 hrs.

Such an aspect of the invention is schematically represented as follows :

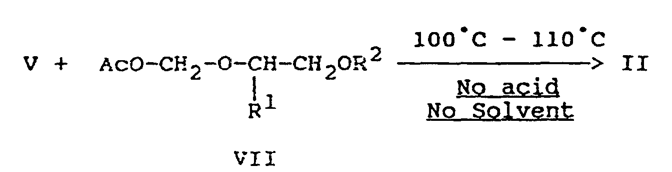

Wherein formula VII is representative of formula VIIa, VIIb and VIIc, with R¹ and R² as defined in formula IIa, IIb and IIc respectively.

In another aspect of the invention there is provided a process for producing practically pure acyclic nucleocides of formula I such as acyclovir and ganciclovir from the compound of formula II obtained as above by basic hydrolysis. Such an aspect of the invention is represented by the following steps :
i) washing the crude intermediate (II) with a solvent selected from methanol, ethanol, iso-propanol, acetone, THF, dioxane, dimethoxyethane, acetonitrile, toluene, benzene, ethyl acetate, dichloromethane or mixture thereof to remove the traces of non-polar impurities.
ii) deprotection of the various functional groups such as esters, or benzyl ethers of the intermediate (II) to yield pure N-9 isomer (I) i.e acyclovir or ganciclovir of extremely high purity.

Other aspects of the invention will become apparent from the description given herein below.

With the objectives of the present invention discussed above in mind, extensive study on the subject made by the present inventors revealed that in the presence of an acid catalyst and a solvent, the alkylation reaction of DAG(Va) and OBDDA, gives significant proportion of N-7 isomer (III) along with other side products and also poor yields of N-9 isomer (II).
Thus, in a homogeneous mixture of DAG(Va), OBDDA, p-toluene sulfonic acid (p-TsOH) and a solvent, the ratio of N-9 to N-7 isomer varies from 2.3 to 2.6. Further, with higher concentration of acid the ratio of N-9/N-7 isomer is observed to come down substantially as is clearly illustrated in Table 1 hereunder.

**TABLE 1**

| Effect of various concentrations of p-TsOH on N-9/N-7 ratio (of II & III) | | | | | | | | |
|---|---|---|---|---|---|---|---|---|
| Reactants : | | | | | | | | |
| Diacetyl guanine(Va) : 10 gms (0.0425 moles) | | | | | | | | |
| OBDDA (VIIa) : 18.7 gms (0.106 moles) | | | | | | | | |
| Reaction Conditions : Reaction mixture heated at 100°C -105°C in a round bottom flask. | | | | | | | | |

| Sr No | Amt of p-TsOH.H₂O used | | | Reaction Time Hr | HPLC monitoring of condensation reaction | | | |
|---|---|---|---|---|---|---|---|---|
| | Gms | moles X 10⁻³ | molar ratio pTsOH /DAG | | N-9 isomer | N-7 isomer | % ratio calculated | |
| | | | | | | | N-9 isomer | N-7 isomer |
| 1 | 0 | 0 | 0 | 70 | 95.60 | 2.90 | 97.05 | 2.94 |
| 2 | 0.05 | 0.265 | 0.0063 | 28 | 92.60 | 3.70 | 96.45 | 3.54 |
| 3 | 0.10 | 0.53 | 0.0125 | 21 | 90.10 | 5.10 | 94.64 | 5.36 |
| 4 | 0.19 | 1.06 | 0.0250 | 15 | 82.90 | 5.70 | 93.56 | 6.43 |
| 5 | 0.40 | 2.12 | 0.050 | 15 | 80.20 | 6.50 | 92.50 | 7.50 |
| 6 | 0.80 | 4.21 | 0.100 | 15 | 77.50 | 7.90 | 90.70 | 9.20 |

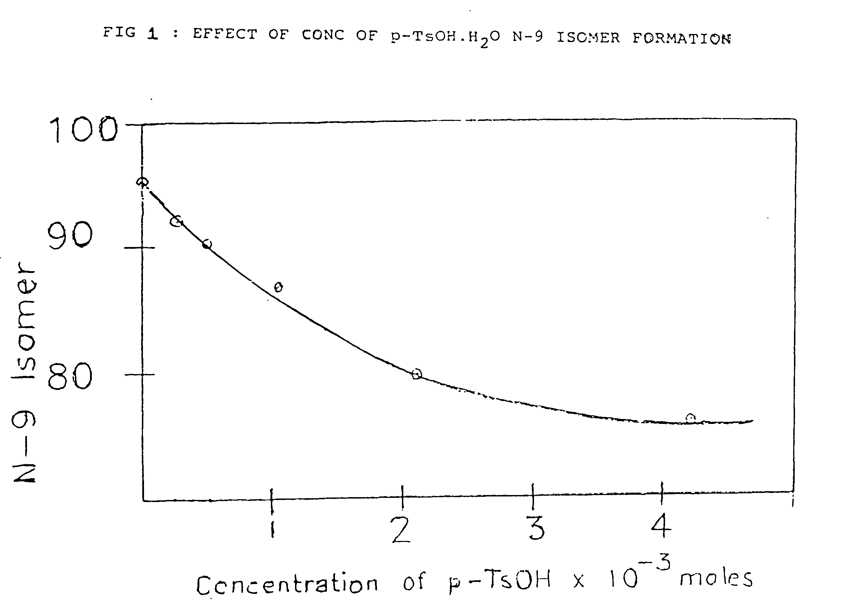

From Table 1, one would observe that with higher concentration of acid, the ratio of N-9/N-7 isomer comes down substantially. The same data is plotted in Figure 1.

The inventors further observed that N²-acetylguanine i.e. N²-monoacetylguanine(MAG, VIa) cannot be alkylated in the absence of acid catalyst by OBDDA. However, surprisingly enough the inventors have identified that the compound of formula V such as diacetylguanine (DAG, Va) can be converted to the intermediate (II) in the presence of OBDDA or the corresponding alkylating agents used to synthesis ganciclovir without the aid of any acid catalyst or solvent only when reacted under specific modified reaction conditions.

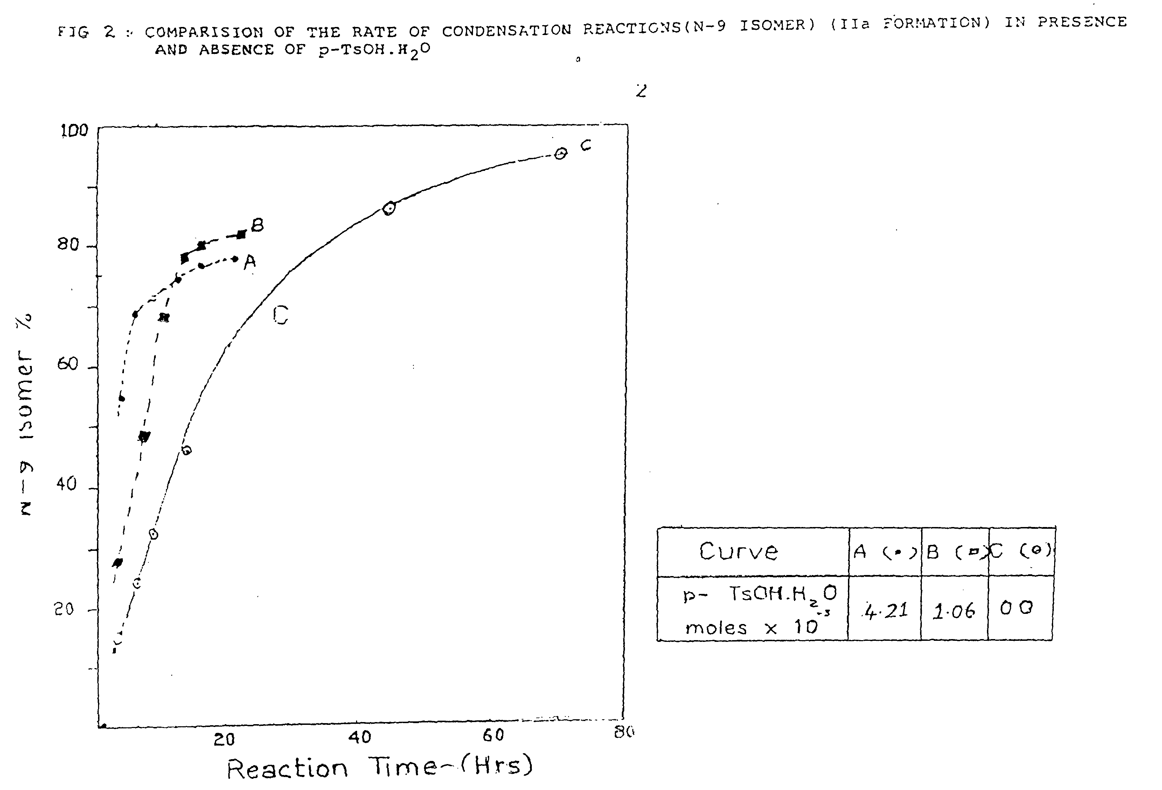

Thus, the present inventors have found that under said specific modified reaction conditions and in the absence of any acid catalyst and solvent although the reaction between DAG (Va) and OBDDA was slower but such conditions provided much purer N-9 isomer (IIa) with very little concomitant formation of the undesired N-7 isomer. Such findings of the invention are illustrated hereunder in Table 2 which indicates the rate of formation of N-9 isomer of formual IIa. using two different concentrations of the acid catalyst viz. p-toluene sulphonic acid and without any such acid. The formation of N-9 isomer was monitered through HPLC analysis and the results are further plotted in figure 2.

**TABLE 2**

| Comparison of rate of formation of N-9 isomer in the presence and absence of p-toluene sulfonic acid.H₂O(p-TsOH.H₂O). | | | | |
|---|---|---|---|---|
| Reactants : | | | | |
| Diacetyl guanine (Va):- 10 gms (0.0425 moles) | | | | |
| OBDDA (VIIa) :- 18.7 gms (0.106 moles) | | | | |
| Reaction Conditions : Reaction mixture heated at 100°C - 105°C, | | | | |

| | | A | B | C |
|---|---|---|---|---|
| P-TsOH.H₂O moles X 10-3 | | 4.21 | 1.06 | 0 |

| Sr No. | Reaction Times Hrs | % N-9 Isomer formed* | | |
|---|---|---|---|---|
| 1 | 03 | 55.20 | 28.50 | 15.1 |
| 2 | 06 | 69.10 | 49.20 | 24.6 |
| 3 | 09 | 70.20 | 69.10 | 31.9 |
| 4 | 12 | 73.10 | 77.20 | 44.16 |
| 5 | 15 | 76.30 | 80.50 | 85.3(44hrs) |
| 6 | 21 | 77.50 | 81.20 | 95.6(70hrs) |

| | | | | |
|---|---|---|---|---|
| * Based on HPLC monitoring of the alkylation step. | | | | |

The above table 2 clearly reveals that the formation of N-9 isomer is more when the alkylation reaction is carried out in the absence of any acid as compared to the use of the acid when the reaction time is allowed to proceed beyond 15 hrs.

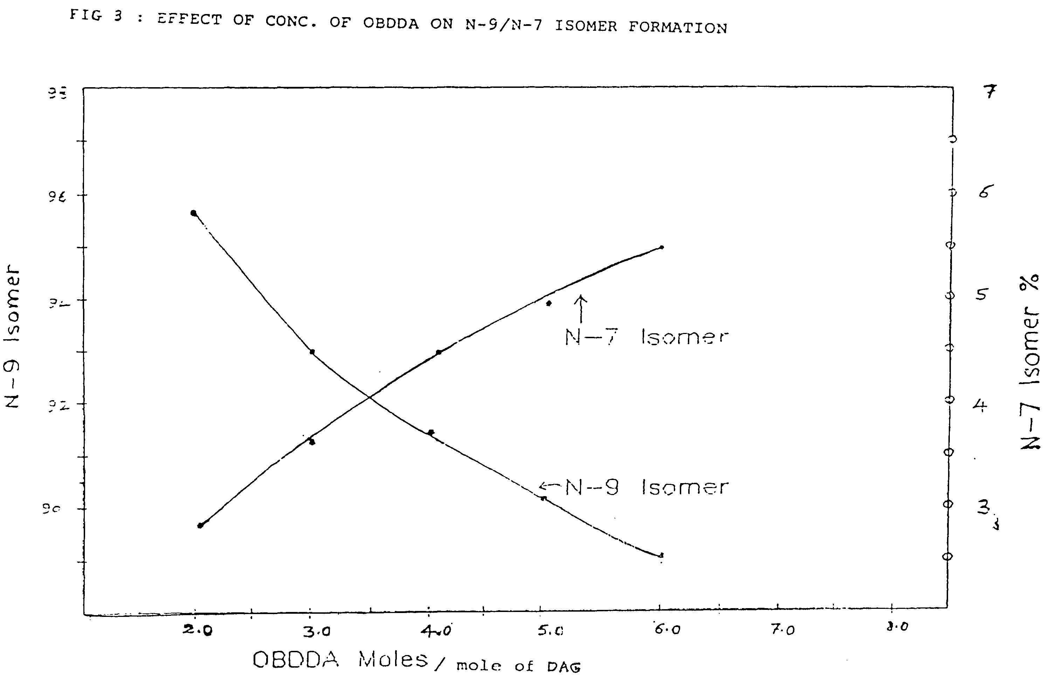

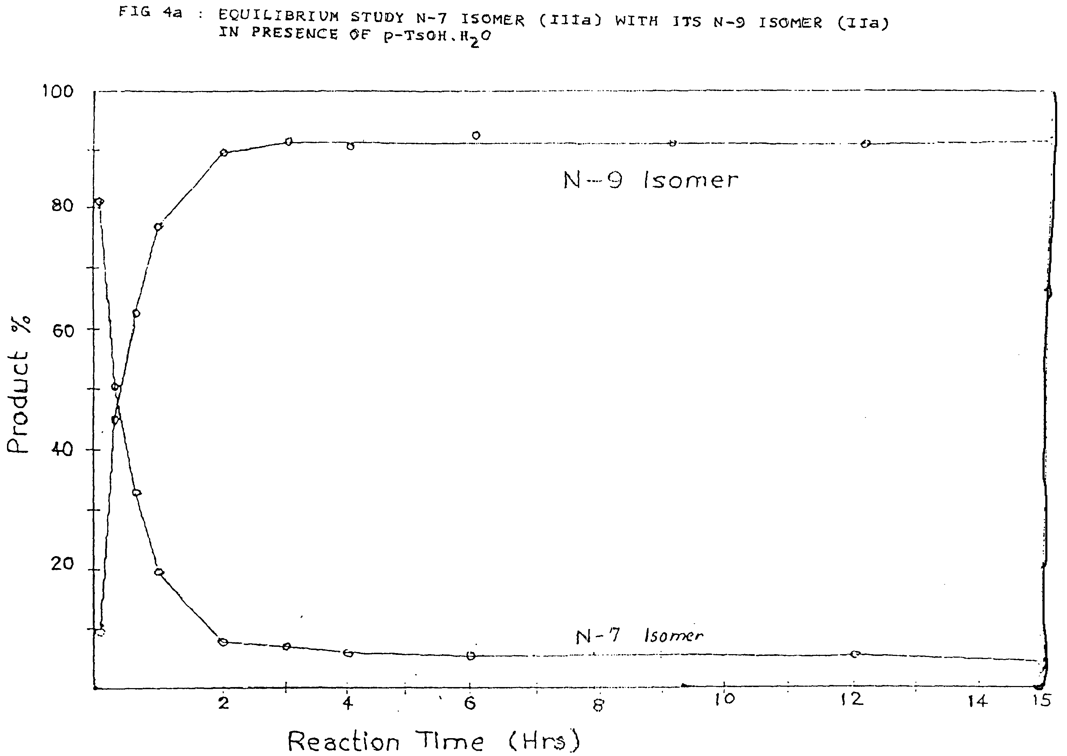

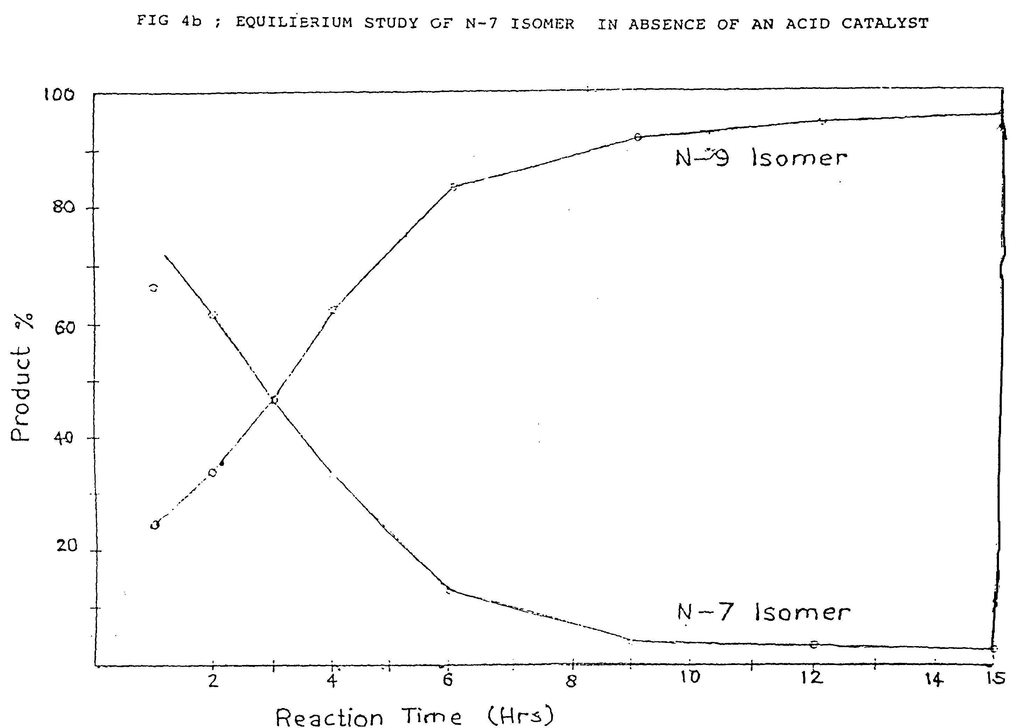

With such findings of favourable yield of N-9 isomer in the absence of any acid catalyst for the alkylating reaction of diacetyl guanine with OBDDA, the dependants of molar proportion of the two reactants on the yield of N-9 isomer of formula II in the absence of acid was determined. The results are reproduced hereunder under Table 3 and further represented in figure 3.

**TABLE 3**

| Effect of concentration of OBDDA on N-9/N-7 isomer formation without acid catalyst | | | | |
|---|---|---|---|---|
| Reaction temperature : 100°C - 105°C | | | | |

| Sr No | OBDDA Used moles/mole DAG | %N-9 isomer* IIa | %N-7 isomer* IIIa | Reaction time Hrs |
|---|---|---|---|---|
| 1 | 2.0 | 95.6 | 2.9 | 75 |
| 2 | 3.0 | 93.0 | 3.6 | 60 |
| 3 | 4.0 | 91.5 | 4.5 | 46 |
| 4 | 5.0 | 90.6 | 4.7 | 38 |
| 5 | 6.0 | 89.0 | 5.4 | 33 |

| | | | | |
|---|---|---|---|---|
| * determined by HPLC monitoring of the reaction. | | | | |

As evident from table 3 above, though with higher proportion of OBDDA the rate of reaction is faster, however yields tend to be lower and the best yield is obtained when approximately two moles of OBDDA per mole of DAG(Va) is used. Thus, when the molar ratio between OBDDA and DAG is 2 and no acid or no solvent is used and the mixture is heated at 100°C - 110°C for 75-80 hour, a mixture of > 95% of N-9 isomer of IIa and < 3% of the corresponding N-7 isomer is obtained.

The observation that the alkylation of DAG(Va) with OBDDA, 1,4-diacetoxy-3-acetoxymethyl-2-oxa-butane or similar alkylating agents, is also a thermodynamically controlled reaction even in the absence of an acid catalyst and a solvent is novel.

The invention accordingly thus further identifies that when the aforesaid N-9 isomer or N-7 isomer is heated at 100°C - 110°C in the presence of OBDDA and in the absence of an acid and solvent, a mixture of N-9 and N-7 isomers is indeed obtained, the former being the major product.

The effect of temperature on the rate of reaction on the ratio of isomer distribution and the yield is quite significant. It has been observed that the yield of N-7 isomer is more at higher temperatures than that at lower temperatures.

Moreover, an acid catalysed reaction at higher temperatures gives lower yields than that of the reaction without any acid catalyst, Higher temperature tends to produce more impurities/side products.

Thus by way of the above the present invention provides that under modified conditions comprising fusion reaction between protected guanine derivatives of structure (V) and alkylating agent (VII) in the molar ratio of 1.5 to 6 at a temperature ranging from 90° - 170°C, preferably between 100°C - 110°C, in absence of solvent or catalyst for 75 - 80 hours it is possible to obtain the compound of formula II and also synthesis of acyclic nucleosides from such compounds of formula II by way of a simple and cost effective process of manufacture.

The objects, advantageous and means of attaining the same as also the scope of the present invention will hereinafter be illustrated in greater detail by way of the following nonlimiting examples. It should be understood that the invention is not intended to be limited to the specific examples.

### EXAMPLES :

### Example 1 : N², N⁹ Diacetyl guanine(DAG, Va):

A mechanically stirred heterogeneous mixture of guanine (22.65 g, 0.15 mole), acetic anhydride (91.8 g, 0.9 mole) and p-toluene sulphonic acid monohydrate (0.70 g, 3.75 X 10⁻³ mole) is heated at 130°C - 135°C for 12 - 14 hours. After the completion of the reaction (monitored by HPLC), the reaction mixture is cooled to room temperature and filtered using cintered funnel. The solid thus obtained is washed with toluene (3 X 25 ml) and dried at 70°C - 80°C under vacuum for 4-5 hours to give 33.1 g (93.9%) diacetyl guanine (DAG), m.p. 300°C(dec)
- PMR(DMSO-d₆)δ ppm :: 8.5(s, 1H, H), 2.85 (s,3H, N⁹-COCH₃),2.2(s,3H,N²-COCH₃)
- IR(KBr)cm⁻¹ :: 3150, 1720, 1705, 1685, 1605, 1528, 1220, 619.
- HPLC Conditions :: Column C₁₈ (reverse phase); eluent, CH₃CN : water ; 20:80 adjusted to pH2 ; detector, UV 254 nm, flow rate 2 ml per minute.

### Example 2: N²-Acetyl-9-[(2-acetoxyethoxy)methyl] guanine (N-9 isomer, IIa) with acid catalyst.

A stirred mixture of DAG (10 g, 0.0425 mole), OBDDA(18.7 g; 0.106 mole) and p-TsOH.H₂O (0.19 g, 0.001mole) was heated in a round bottom flask at 105°C - 110°C for 15 hours. The reaction mixture was concentrated under vacuum and the residue was column chromatographed on SiO₂ column using CH₂Cl₂-CH₃OH (60:40 v/v) solvent system to give the desired N-9 isomer in 85-86% isolated yields, m.p. 189°C - 190°C.
- PMR(DMSO-d₆)δ ppm :: 12.1 (bs, 1H, HNCOCH₃) 11.85 (bs, 1H, NH), 8.2 (s, 1H, H), 5.5 (s, 2H, NCH₂O), 4.15 (m, 2H, OCH₂), 3.7 (m, 2H, OCH₂), 2.2 (s, 3H, NCOCH₃), 1.95 (s, 3H, NCOCH₃).

### Example 3 : N²-Acetyl-9-[(2-acetoxyethoxy)methyl] guanine (N-9 isomer IIa) without acid catalyst

Diacetylguanine (DAG)(10 g, 0.0425 mole) and OBDDA (18.7 g, 0.106 mole) is heated in a round bottom flask at 105°C - 110°C under continuous stirring for 75 - 80 hours. After almost complete conversion of DAG, the excess of OBDDA was distilled out and the residue was heated with a mixture of toluene : methanol (25 : 75, 25 ml) at 45° - 50°C for 30 minutes and the solid was collected by filtration at 5°C - 10°C to yield 98.8 % pure (by HPLC) N-9 isomer in 91.0% isolated yield.

### Example 4 : N²-Acetyl-9-[(2-acetoxyethoxy)methyl] guanine (N=9 isomer, IIa):

A stirred mixture of DAG (10 g, 0.042 mole) and OBDDA (18.7 g; 0.106 mole) was heated in a round bottom flask at 105°C - 110°C for 80 hours. The reaction mixture was concentrated under vacuum and the residue was column chromatographed on SiO₂ column using CH₂Cl₂ : MeOH (6:4 v/v) to give the desired N-9 isomer in ≥ 94% isolated yields, m.p. 189°C - 190°C.

### Example 5: N²-Acetyl-9-[(2-acetoxyethoxy)methyl] guanine (N-9 isomer, IIa):

A stirred mixture of DAG (10 g, 0.0425 mole), OBDDA (44.9 g; 0.255 mole) and p-TsOH.H₂O (0.19 g, 0.001 mole) was heated in a round bottom flask at 100°C - 105°C for 20 hours. Excess OBDDA was removed under vacuum and the residue thus obtained was diluted with toluene (50 ml) and heated at 100°C for 2 hours. The reaction mixture is then cooled at 50°C and filtered to get 11.30 g (87%) crude product.

### Purification of crude product

A suspension of the above obtained crude N-9 isomer (11.30 g) in toluene : isopropyl alcohol (1:1, 50 ml) was heated in round bottom flask at 75°C - 80°C for 2 hours. The reaction mixture is then cooled to 0°C - 5°C, maintained at the same temperature for 1 hour and filtered to get 10.6 g (80%) N-9 isomer of more than 99% HPLC purity.

### Example 6 : N²-acetyl-9[(2-acetoxyethoxy)methyl] guanine : Effect of concentration of acid catalyst on the rate of the reaction

A suspension of DAG (10g, 0.0425 moles) in OBDDA (18.7 g, 0.106 moles) was heated in the presence of different amounts of TsOH.H₂O and also in the absence of the latter at 100°C - 105°C. Samples were withdrawn at regular time intervals and analysed by HPLC. The data is presented in Table II.

### Example 7 : 9-[(2-Hydroxyethoxy)methyl] guanine (Acyclovir):

**To a solution of NaOH pellets (3.8 g, 0.097 mole)** in water (100 ml) is added N-9 isomer (10 g, 0.323 mole) at room temperature. The reaction mixture is heated at 85 C - 95°C for 3 hours. After bringing the temperature down to room temperaturethe pH of the clear solution is adjusted to pH 7 using 35% HCl and filtered to yield ≥95% of the product of very high quality, m.p. 253°C
PMR(DMSO-d₆)*δ* ppm :10.7 (s, 1H, NH), 7.85 (s, 1H, H), 6.51 (s, 2H, N-CH₂-O) 5.3 (s, 2H, NH₂); 4.70 (m, 1H, OH), 3.45 (m, 2H, OCH₂), 3.3 (m, 2H, OCH₂)

### Example 8 : N²-Acetyl-9-[(1,3-bis(benzyloxy)-2-propoxy) methyl] guanine (N-9 isomer; II_{b}) :

A mixture of DAG (5 g, 0.021 mole), 2-O-(acetoxymethyl)-1,3-di-O-benzylglycerol (10.9 g, 0.032 mole) was heated in a round bottom flask under stirring at 110°C - 115°C for 75 - 80 hours. The reaction mixture was cooled to room temperature and extracted with hexane (3 X 15 ml). The residue thus obtained was column chromatographed on SiO₂ column using ethylacetate : hexane (50:50v/v) solvent system to give the desired N-9 isomer (m.p 147°C) in 68.7% yield. The yield of the N-7 isomer (m.p 133°C - 134°C) obtained from column was found to be 14.86%.
PMR (DMSO-d₆-ppm (N-9 isomer) : 8.13 (s, H, H-8), 7.35 (m, 10H, ArH), 5.59 (s, 2H, H-1'); 4.41 (s, 4H, benzylic), 4.05 (m, 1H, H-4'), 3.41 (m, 4H, H-3' & H-5'); 2.18(s, 3H, CH₃)
PMR (DMSO d₆ ppm (N-7 isomer): 8.34 (s, H, H-8), 7.35 (m, 10H, ArH), 5.80 (s, 2H, H-1'); 4.42 (s, 4H, benzylic), 4.14 (m, 1H, H-4'), 3.48 (m, 4H, H -3' & H-5'); 2.19(s, 3H, CH₃)

### Example 9 : 9-[(1,3-dihydroxy-2-propoxy)methyl]guanine (ganciclovir):

The synthesis of Ganciclovir starting from N²-acetyl-9 [1,3-bis (benzyloxy)-2-propoxy) methyl] guanine (N-9 isomer, II_{b}) obtained from example. No. 11 was carried out following the reported conditions (J C Martin et al J. Med. Chem. 1983, 26, 759-761) to get the desired product of high purity in 76% isolated yield, m.p > 300°C.
PMR (DMSO-d₆) δ : 10.64(bs, 1H, NH), 7.81(s 1H, H-8), 6.5 (s, 2H, NH₂), 5.44(s, 2H, H-1'), 4.63 (p, J = 6 Hz, 1H, H-4'), 3.35 (m, H-3' & H-5')

## Claims

1. A regiospecific process for the synthesis of compounds of formula II, 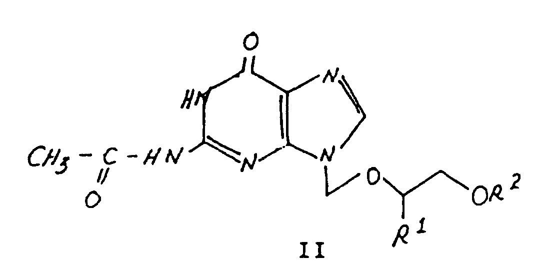 wherein
IIa : R¹ = H, R² = COCH₃
IIb : R¹ = CH₂OCH₂Ph, R² = CH₂Ph
IIc : R¹ = CH₂OCOCH₃, R² = COCH₃
which comprises reacting a substituted guanine derivative of formula V, 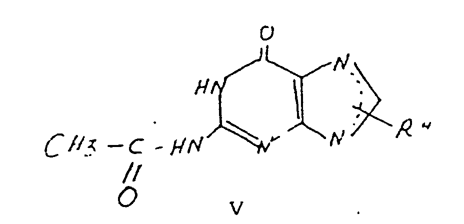 wherein 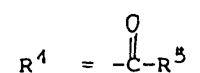 where R⁵ = methyl, ethyl, isopropyl, phenyl with alkylating agent of formula VII, 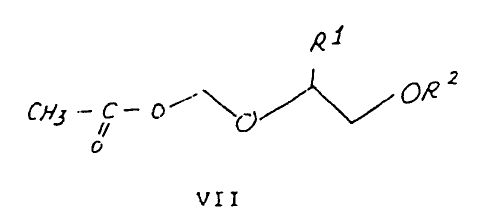 wherein R¹ and R² is as defined in formula II, without the presence of any acid catalyst and/or solvent under modified conditions comprising carrying out said reaction between protected guanine derivative and said alkylating agent in the molar ratio of 1.5 to 6.0 at a temperature ranging from 90°-170°C for a period of 75-80 hrs.

2. A process of claim 1 wherein the compound of formula II is N²-acetyl-9-[(2-acetoxyethoxy)methyl]guanine (IIa) obtained by reacting diacetylguanine (DAG) of formula V with 2-oxa-1,4-butandiol diacetate (OBDDA) of formula VII.

3. A process of claim 1 wherein the compound of formula II is N²-acetyl-9-[1,3-bis(benzyloxy)-2-propoxy)methyl]guanine(IIb) obtained by reacting diacetylguanine (DAG, Va) with 1,4-dibenzyloxy-3-acetoxymethyl-2-oxabutane of formula VII.

4. A process of any one of claims 1 to 3, wherein the compound of formula II is purified by washing with solvents such as methanol, ethanol, iso-propanol, dioxane, THF, 1,2-dimethyoxyethane, acetonitrile, toluene, benzene, dichloromethane, ethyl acetate or mixture thereof.

5. A process of any one of claims 1 to 4 which additionally comprises the functional group deprotection of the compound of formula II to regiospecifically synthesise an acyclic nucleoside of formula I 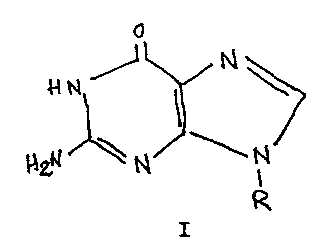 Wherein
Ia : R = -CH₂-O-CH₂CH₂OH
Ib : R = CH₂-O-CH(CH₂OH)CH₂OH
by functional group deprotection of the intermediate II stated in claim 1.

6. A process of claim 5 wherein the said deprotection of functional groups of the intermediates IIa and IIc stated in claim 1 is carried out by treating with an aqueous alkali.

7. A process of claim 6 wherein the said aqueous alkali is selected from sodium hydroxide, sodium carbonate, ammonium hydroxide, potassium carbonate, sodium bicarbonate.

8. A regiospecific process of any one of claims 1 to 4 wherein the molar ratio of guanine derivative to alkylating agent is in the range 1.5 to 2.5.

9. A regiospecific process of any one of claims 1 to 4 and 8 wherein the reaction is carried out at a temperature of between 100°C and 110°C.

## Patentansprüche

1. Regiospezifisches Verfahren zur Synthese von Verbindungen der Formel II 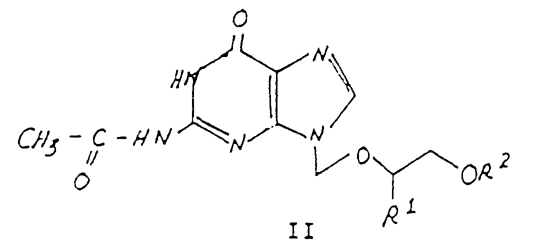 worin
IIa : R¹ = H, R² = COCH₃
IIb : R¹ = CH₂OCH₂Ph, R² = CH₂Ph
IIc : R¹ = CH₂OCOCH₃, R² = COCH₃
sind, umfassend das Umsetzen eines substituierten Guaninderivats der Formel V 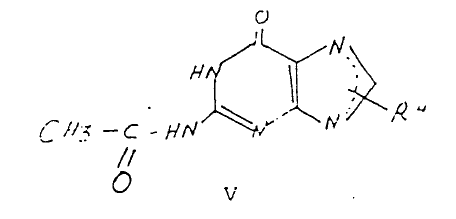 worin 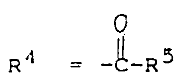 ist, worin R⁵ = Methyl, Ethyl, Isopropyl oder Phenyl ist, mit einem Alkylierungsmittel der Formel VII 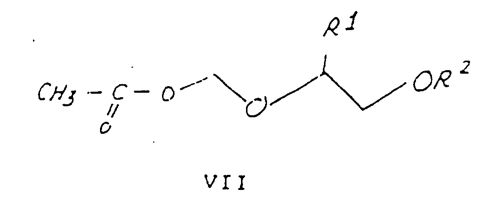 worin R¹ und R² wie in Formel II definiert sind, ohne die Gegenwart jeglichen Säurekatalysators und/oder Lösungsmittels unter modifizierten Bedingungen, umfassend die Durchführung der Reaktion zwischen geschütztem Guaninderivat und dem Alkylierungsmittel in einem Molverhältnis von 1,5 bis 6,0 bei einer Temperatur im Bereich von 90 bis 170 °C über einen Zeitraum von 75 bis 80 Stunden.

2. Verfahren nach Anspruch 1, worin die Verbindung der Formel II N²-Acetyl-9-[(2-acetoxyethoxy)methyl]guanin (IIa), erhalten durch Umsetzen von Diacetylguanin (DAG) der Formel V mit 2-Oxa-1,4-butandioldiacetat (OBDDA) der Formel VII, ist.

3. Verfahren nach Anspruch 1, worin die Verbindung der Formel II N²-Acetyl-9-[1,3-bis(benzyloxy)-2-propoxy)methyl]guanin (IIb), erhalten durch Umsetzen von Diacetylguanin (DAG, Va) mit 1,4-Dibenzyloxy-3-acetoxymethyl-2-oxabutan der Formel VII, ist.

4. Verfahren nach einem der Ansprüche 1 bis 3, worin die Verbindung der Formel II durch Waschen mit Lösungsmitteln, wie z.B. Methanol, Ethanol, Isopropanol, Dioxan, THF, 1,2-Dimethoxyethan, Acetonitril, Toluol, Benzol, Dichlormethan, Ethylacetat oder einem Gemisch davon, gereinigt wird.

5. Verfahren nach einem der Ansprüche 1 bis 4, weiters umfassend die Entfernung der Schutzgruppe von der funktionellen Gruppe der Verbindung der Formel II, um ein azyklisches Nukleosid der Formel I 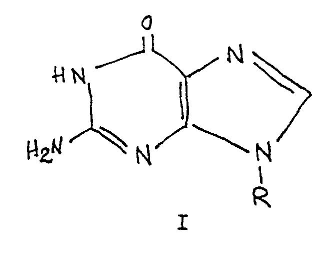 worin
Ia : R = -CH₂-O-CH₂CH₂OH
Ib : = CH₂-O-CH(CH₂OH)CH₂OH
sind, durch Entfernung der Schutzgruppe von der funktionellen Gruppe des Zwischenprodukts II aus Anspruch 1 regiospezifisch zu synthetisieren.

6. Verfahren nach Anspruch 5, worin die Entfernung der Schutzgruppe von der funktionellen Gruppe der Zwischenprodukte IIa und IIc aus Anspruch 1 durch Behandlung mit einer wässrigen Base erfolgt.

7. Verfahren nach Anspruch 6, worin die wässrige Base aus Natriumhydroxid, Natriumcarbonat, Ammoniumhydroxid, Kaliumcarbonat und Natriumbicarbonat ausgewählt ist.

8. Regiospezifisches Verfahren nach einem der Ansprüche 1 bis 4, worin das Molverhältnis zwischen Guaninderivat und Alkylierungsmittel im Bereich von 1,5 bis 2,5 liegt.

9. Regiospezifisches Verfahren nach einem der Ansprüche 1 bis 4 oder 8, worin die Reaktion bei einer Temperatur zwischen 100 und 110 °C durchgeführt wird.

## Revendications

1. Procédé régiospécifique pour la synthèse de composés de la formule II, 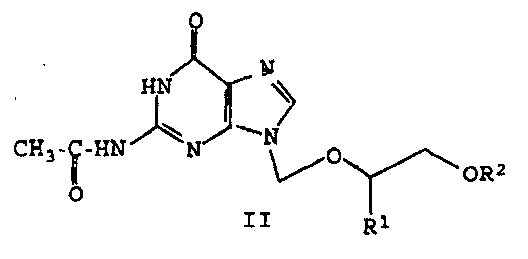 où
IIa : R¹ = H, R² = COCH₃
IIb : R¹ = CH₂OCH₂Ph, R² = CH₂Ph
IIc : R¹ = CH₂OCOCH₃, R² = COCH₃
qui comprend la réaction d'un dérivé de guanine substituée de formule V, 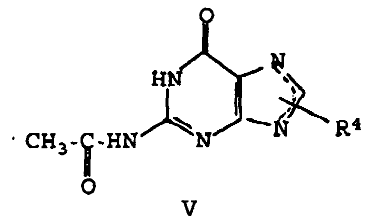 où 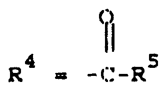 où R⁵ = méthyle, éthyle, isopropyle, phényle avec un agent alkylant de la formule VII, 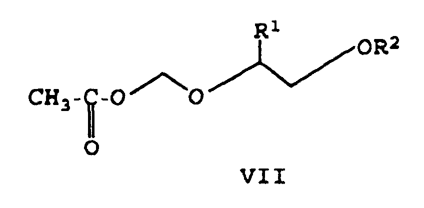 où R¹ et R² est tel que défini à la formule II, sans la présence d'un catalyseur acide et/ou d'un solvant en conditions modifiées consistant à effectuer ladite réaction entre le dérivé de guanine protégée et ledit agent alkylant au rapport molaire de 1,5 à 6,0 à une température de 90°-170°C pendant une période de 75-80 heures.

2. Procédé de la revendication 1 où le composé de formule II est la N²-acétyl-9-[(2-acétoxyéthoxy)méthyl]guanine (IIa) obtenue par réaction de diacétylguanine (DAG) de formule V avec du 2-oxa-1,4-butanediol diacétate (OBDDA) de formule VII.

3. Procédé de la revendication 1 où le composé de formule II est la N²-acétyl-9-[1,3-bis(benzyloxy)-2-propoxy)méthyl]guanine (IIb) obtenue par réaction de diacétylguanine (DAG, Va) avec du 1,4-dibenzyloxy-3-acétoxyméthyl-2-oxabutane de formule VII.

4. Procédé selon l'une quelconque des revendications 1 à 3, où le composé de formule II est purifié par lavage avec des solvants tels que méthanol, éthanol, isopropanol, dioxane, THF, 1,2-diméthoxyéthane, acétonitrile, toluène, benzène, dichlorométhane, acétate d'éthyle ou leurs mélanges.

5. Procédé selon l'une quelconque des revendications 1 à. 4 qui comprend additionnellement la suppression de la protection des groupes fonctionnels des composés de formule II pour synthétiser régiospécifiquement un nucléoside acyclique de formule I 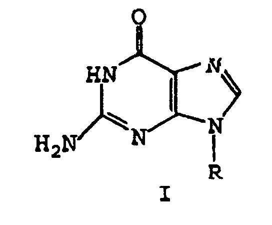 où
Ia : R = -CH₂-O-CH₂CH₂OH
Ib : R = CH₂-O-CH(CH₂OH)CH₂OH
par suppression de la protection des groupes fonctionnels de l'intermédiaire II indiqué à la revendication 1.

6. Procédé de la revendication 5 où ladite suppression de la protection des groupes fonctionnels des intermédiaires IIa et IIc indiqués à la revendication 1 est effectuée par traitement avec un alcali aqueux.

7. Procédé de la revendication 6 où ledit alcali aqueux est sélectionné parmi l'hydroxyde de sodium, le carbonate de sodium, l'hydroxyde d'ammonium, le carbonate de potassium, le bicarbonate de soude.

8. Procédé régiospécifique selon l'une quelconque des revendications 1 à 4 où le rapport molaire du dérivé de guanine à l'agent alkylant est dans la gamme de 1,5 à 2,5.

9. Procédé régiospécifique selon l'une quelconque des revendications 1 à 4 et 8 où la réaction est effectuée à une température comprise entre 100°C et 110°C.
